# EUROPEAN PATENT APPLICATION

(11) **EP 2 626 052 A1**
(43) Date of publication of application: **14.08.2013**
(21) Application number: 12736793.6
(22) Date of filing: 20.01.2012
(51) Int. Cl.: A61H 33/02, A61M 15/00, A61M 16/12

(54) **GAS MIST INHALER**

(30) Priority: 21.01.2011 JP 2011010647
(71) Applicant: Nakamura, Shoichi, Higashichikuma-gun Nagano 399-7502 (JP); ACP Japan Co. Ltd., Tokyo 113-0033 (JP)
(72) Inventor: The designation of the inventor has not yet been filed
(74) Representative: Gill, David Alan
(86) International application number: PCT/JP2012/051247
(87) International publication number: WO 2012/099250

(57) **Abstract**

The invention is to offer a gas mist inhaler which makes only one part of the gas mist inhaler disposable for ensuring hygiene as well as realizing economization. The gas mist inhaler comprises a gas supply means 30 of supplying oxygen, carbon dioxide, or a mixed gas of oxygen and carbon dioxide; a gas mist generating means 40 which has a connecting portion 45 connected to the gas supply means 30, a liquid storage of storing a liquid, a nozzle supplied with the gas, a liquid suction pipe of sending the liquid to the tip of the nozzle, a collision member of colliding the liquid blown up by a gas stream from the nozzle therewith to convert it into a gas mist, a cylindrical gas inlet leading the gas till the upper portion of the nozzle, and a donut-shaped gas mist outlet collecting and discharging the gas mist; and an inhalation member 50 connected to the gas mist generating member 40 and having an inhalation port 53 for enabling to inhale the gas mist into a living organism, and wherein the gas mist generating means 40 is structured such that at least the liquid storage is made replaceable with others, and is disposable.

## Description

### TECHNICAL FIELD

The present invention relates to a gas mist inhaler for carrying out oral inhalation of a gas mist into a living organism, the gas mist having been prepared by pulverizing and dissolving oxygen, carbon dioxide or a mixed gas of oxygen and carbon dioxide and liquid of such as medicines.

### BACKGROUND OF THE INVENTION

Conventionally, it has been known that carbon dioxide (carbonic acid anhydride: CO₂) has two properties of being not only soluble in water (water-soluble) but also soluble in fat (fat-soluble) and, owing to having both properties, when only contacting to a skin and a mucous membrane of a living organism which are like as mixed with water and fat, carbon dioxide penetrates under a subcutaneous tissue and expands blood vessels around the penetrated parts, and it works to improve the blood circulation. By this action of accelerating the blood circulation, it displays various physiological effects such as dropping of blood pressure, improving of metabolism or accelerating to remove pain substance or waste product. Further, it has also anti-inflammation and anti-bacterial function. Therefore, carbon dioxide has recently been given attentions also from viewpoints of improving health or beauty other than the purpose of medical cares.

In the tissue of the living organism, carbon dioxide works to release oxygen having been carried in combination with hemoglobin in a red blood cell. Around parts at the high density of carbon dioxide, the red blood cell releases more oxygen. Thus, supply of oxygen to cells by the red blood cell is mainly controlled by carbon dioxide. In short, being without carbon dioxide, hemoglobin remains as having been combined with oxygen and the cell becomes unable to receive oxygen. Carbon dioxide seems to be waste products resulted from action of the cell, however, as is seen, it plays in fact very important roles in the living organism.

Further, recently, oxygen of the high density has also widely been known as effective over activity of metabolism, acceleration of blood circulation, fatigue recovery, or stability of blood pressure. Other than them, oxygen has effects of disinfection or sterilization by oxidation.

By the way, for easing a symptom of disease in a respiratory system such as asthma or allergic rhinitis, oral or nasogastric inhalation of medicines or steam using an inhaler or a nose spray have been till now carried out.

A nasogastric vaccine (collunarium vaccine of spray system) has recently developed for forming a mucous immunity against an influenza virus, and its high effect has been given attention. The nasogastric vaccine is generally higher in effects of preventing crises of influenza than the vaccine injected under the skin, and has a merit of effectively working against various virus stocks differently from injection types. In addition, also in prevention or cure to the influenza, an antiviral drug of inhalation dosage has been developed.

Therefore, an inventor of this invention has developed a gas mist inhaler which efficiently dissolves carbon dioxide or oxygen in medicines as above mentioned and effectively gives physiological actions to the living organism in addition to the medicines.

### SUMMARY OF THE INVENTION

### PROBLEMS THAT THE INVENTION IS TO SOLVE

For ensuring hygiene of the inhalers as above mentioned, desirably, parts of generating the gas mist are made disposable. However, if disposable parts are large in size, a cost becomes high and futility increases.

Accordingly, in view of the above mentioned circumstances, it is an object of the invention to provide such a gas mist inhaler, only one part of which is made disposable for ensuring hygiene and can realize reduction in costs.

### MEANS FOR SOLVING THE PROBLEMS

For solving the above mentioned problems, the invention is to provide a gas mist inhaler which comprises a gas supply means of supplying oxygen, carbon dioxide, or a mixed gas (called as "gas" hereafter) of oxygen and carbon dioxide; a gas mist generating means which has a connecting portion connected to the gas supply means, a liquid storage of storing a liquid, a nozzle supplied with the gas, a liquid suction pipe of sending the liquid to the tip of the nozzle, a collision member of colliding the liquid blown up by a gas stream from the nozzle therewith to convert it into a mist (called as "gas mist" hereafter) by pulverizing and dissolving these liquid and gas, a cylindrical gas inlet supplied with the gas and leading the gas till the upper portion of the nozzle, and a donut-shaped gas mist outlet collecting and discharging the gas mist; and an inhalation member connected to the gas mist generating member and having an inhalation port for enabling to inhale the gas mist into a living organism, and wherein the gas mist generating means is characterized in that at least the liquid storage is replaced by another liquid storage.

By the way, the invention refers it as "pulverizing and dissolving" to pulverize the liquid into fine liquid drops, and cause to contact and mix with gas (oxygen or carbon dioxide, or the mixed gas of oxygen and carbon dioxide).

Herein, preferably, the gas mist generating means has an air hole for taking in outside air, otherwise it is also sufficient for the inhalation member to have a further opening for taking in outside air.

The gas supply means is desirably a gas bomb of a cartridge system. Further, the gas supply means may have any one or plurality of a gas supply time setting portion, a gas supply pressure adjusting portion and a gas mixing ratio setting portion.

In addition, the gas mist generating means also may supply the gas mist into the plural inhalation members.

Next, it is best that the above mentioned liquid is any one or plural combination of water, ionic water, ozone water, physiological salt solution, purified water, or sterilized and purified water. And it is desirable to further contain any one or plural combination of menthol, vitamin E, vitamin C derivative, retinol, anesthetic agent, cyclodextrin, photo catalyst, complex of photo catalyst and apatite, hyaluronic acid, coenzyme Q10, seed oil, propolis, ethanol, chlorhexidine gluconate, amphoteric surface active agent, benzalkonium chloride, alkyl diamino etherglycine acetate, sodium hypochlorite, peracetic acid, sodium sesquicarbonate, silica, povidone-iodine, sodium hydrogen carbonate, carbonate spring agent of high density, anti-allergic agent, anti-inflammatory agent, anti-febrile agent, anti-fungus agent, anti-influenza virus agent, influenza vaccine, steroid agent, anti-cancer agent, or anti-hypertensive agent.

A size of the mist supplied from the gas mist generating means into the inhalation member is suitably not larger than 10 µm.

Desirably, the gas mist generating means has a gas mist supply pipe for supplying the gas mist into the inhalation member, and this gas mist supply pipe is preferably furnished with a filter for removing liquid drops attached to the inside of the pipe. Still further, the gas mist supply pipe is suitably composed of a cornice shaped pipe over a whole or at one part of the gas mist supply pipe . In addition, this gas mist supply pipe is provided with a check valve.

Preferably, the removable part of the gas mist generating means is in advance sterilized.

### EFFECTS OF THE INVENTION

According to the gas mist inhaler of the invention, by making one part of the gas mist generator replaceable (disposable), it enables to pay attention to hygiene together with reduction in costs. Thereby, with the simple structure, by the physiological action of the gas mist, not only permeating a liquid medicine into the upper and lower airways of the living organism, but also activating a blood flow around a diseased part, the invention can display effects such as flourishing the blood flow of the disease, rapidly moderating an inflammation or increasing immunological force.

### BRIEF DESCRIPTION OF THE DRAWINGS

[FIG. 1] A generally schematic view of the gas mist inhaler depending on the first embodiment of the invention;
[FIG. 2] A typical and cross sectional view showing the structure of the gas mist generator in the gas mist inhaler of FIG. 1;
[FIG. 3] A perspective view, partially in section, of the generator main body in the gas mist generator shown in FIG. 1;
[FIGs. 4A-4B] Typical cross sectional views showing embodiments of sealing the generator main body in the gas mist generator in FIG. 1;
[FIG. 5] A perspective view under a condition of looking the cover part member from the lower part in the gas mist generator shown in FIG. 1,
[FIG. 6] A partially enlarged cross sectional view of the generator main body in the gas mist inhaler in FIG. 1;
[FIGs. 7A-7B] Typical views showing examples of the inhalation members in the gas mist inhaler of the invention;
[FIG. 8] A typical view showing the example of the gas mist supply pipe connecting the gas mist generator to the inhalation member of the invention;
[FIG. 9] A generally schematic view of the gas mist inhaler depending on a second embodiment of the invention; and
[FIG. 10] A generally schematic view of the gas mist inhaler depending on a third embodiment of the invention.

### EMBODIMENTS FOR PRACTICING THE INVENTION

In the following description, explanations will be made to embodiments of this invention, referring to the attached drawings.

### [First Embodiment]

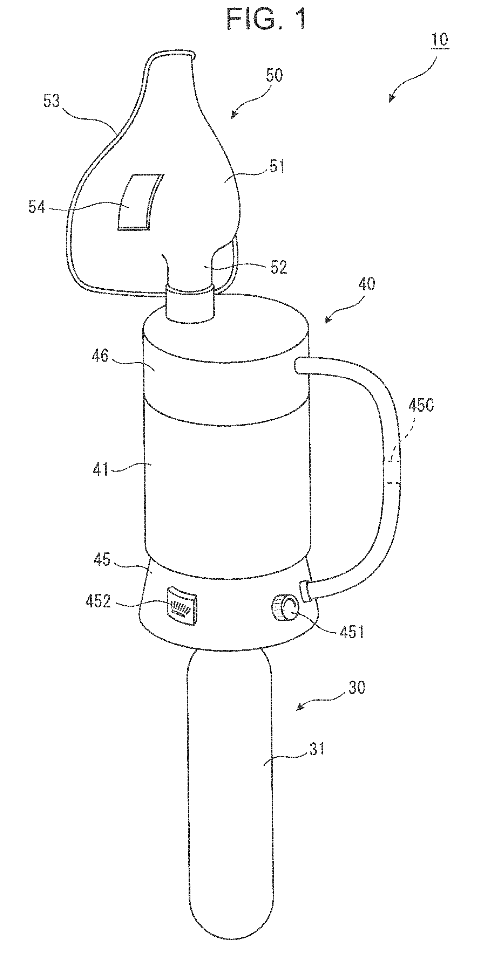

FIG. 1 is the generally schematic view of the gas mist inhaler depending on the first embodiment of the invention.

As showing in FIG. 1, the gas mist inhaler 10 of the present embodiment has a gas bomb 31 as a gas supply means 30 of supplying oxygen, carbon dioxide or a mixed gas (called briefly as "gas" hereafter) of oxygen and carbon dioxide, a gas mist generator 40 as a gas mist generating means and an inhalation mask 51 as an inhalation member 50.

The gas bomb 31 of supplying oxygen, carbon dioxide or the mixed gas of oxygen and carbon dioxide into the gas mist generator 40 employs a small sized cartridge system in the present embodiment, paying attention to portability. This small sized gas bomb 31 is, as shown in FIG. 1, attached to a gas supply means connecting portion 45 of the gas mist generator 40 for supplying gas into the gas mist generator 40 at predetermined pressure.

The gas mist generator 40 stores inside a liquid, generates a gas mist prepared by pulverizing and dissolving this liquid and gas by high speed flowing of the gas supplied from the gas bomb 31, and supplies the gas mist into the inhalation mask 51.

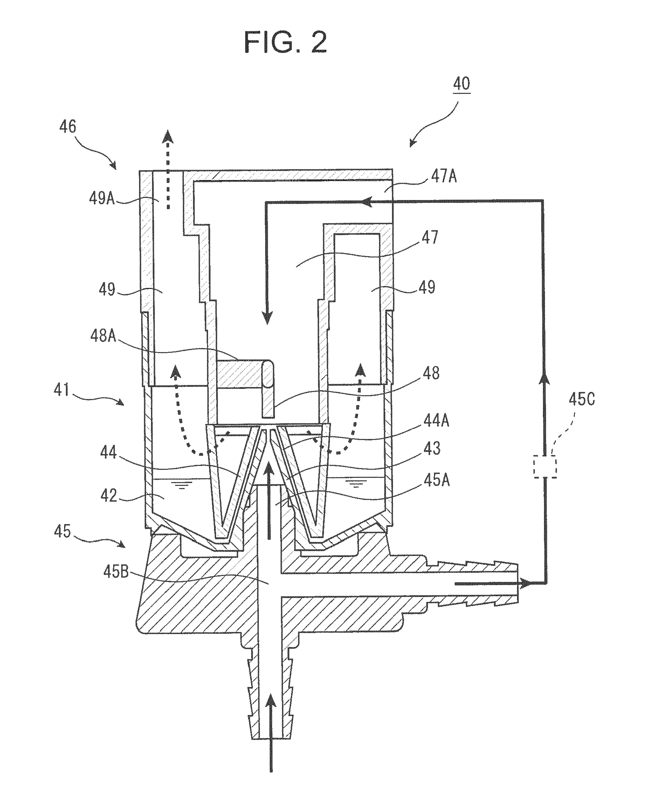

FIG. 2 is the typical and cross sectional view showing the structure of the gas mist generator 40. As shown in FIG. 2, the gas mist generator 40 is composed with a generator main body 41, the a gas supply means connecting portion 45 and a cover part 46.

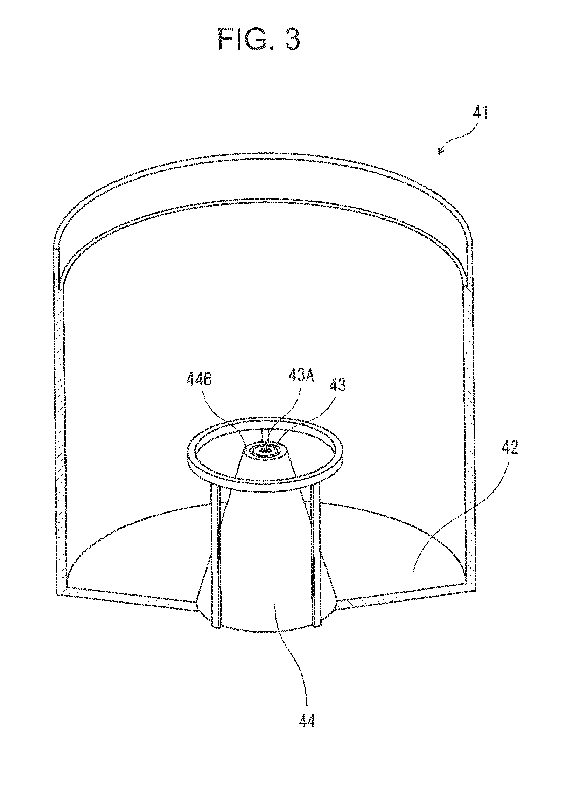

FIG. 3 is the perspective view, partially in section, of the generator main body 41. As shown therein, the generator main body 41 is provided with a liquid storage 42 for storing the liquid, a nozzle 43 of discharging the gas supplied from the gas bomb 31 from a point open 43A, and a liquid suction pipe 44 of sucking up the liquid stored in the liquid storage 42 until the front end of the nozzle 43.

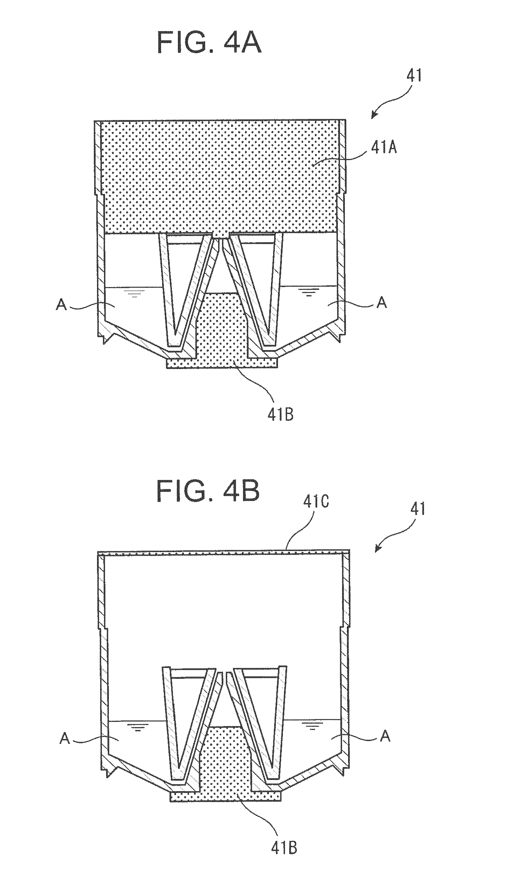

The liquid storage 42 is, as shown in FIGs. 2 and 3, formed with the inside of the bottom of the generator main body 41 and the inside of one part of its side wall. This liquid storage 42 is stored with a predetermined liquid previously when building a setting-up stage. Under conditions of having stored the liquid, the generator main body 41 is sealed as shown in FIGs. 4A-4B. FIGs. 4A-4B are the typical views showing the embodiments of sealing the generator main body 41. FIG. 4A shows an example of sealing the generator main body 41 at its upper and lower parts with plugs 41A, 41B made of elastic members such as a rubber. FIG. 4B shows an example of sealing the generator main body 41 at its upper part with a film 41C made of an aluminum or a plastic and sealing the generator main body 41 at its lower part with a plug 41B made of an elastic member.

Thus, the invention makes the liquid storage 42 removable in the gas mist generator 40 for replacing it with another liquid storage 42 so that disposable parts are made less to restrain costs . Further, the generator main body 41 is offered as being sealed, and by making disposable at any time, hygiene can be thus maintained. It is possible thereby to omit a structure of supplying the liquid such as medicines into the liquid storage 42, and realize reduction in size and in cost. By the way, this removable generator main body 41 is preferably sterilized in a manufacturing stage, Further, non-displaceable parts are also dealt with sterilization treatment prior to use.

Herein, for the liquid stored in the liquid storage 42, it is preferable to employ water, ionic water, ozone water physiological salt solution, purified water or sterilized and purified water. Further, these liquids are sufficient to contain medicines useful to users' diseases or symptom. As the medicines, for example, listed are anti-allergic agent, anti-inflammatory agent, anti-febrile agent, anti-fungus agent, anti-influenza virus agent, influenza vaccine, steroid agent, anti-cancer agent, or anti-hypertensive agent. Further, these liquids are further possible to generate synergistic effects by coupling with a gas physiological action with single or plurality of menthol having a cooling action; vitamin E accelerating circulation of the blood; vitamin C derivative easily to be absorbed to a skin tissue and having a skin beautifying effect; retinol normalizing a skin heratinizing action and protecting the mucous membrane; anesthetic agent moderating irritation to the mucous membrane; cyclodextrin removing odor; photocatalysis or a complex of photocatalysis and apatite having disinfection and anti-phlogistic; hyaluronic acid having excellent water holding capacity and a skin moisture retention effect; coenzyme Q10 activating cells and heightening immunization; a seed oil containing anti-oxidation and much nutrient; or propolis having anti-oxidation, anti-fungus, ant-inflummatory agent, pain-killing, anesthetic, and immunity. Otherwise, the liquids may be added with ethanol, gluconic acid chlorohexizine, amphoteric surface active agent, benzalkonium chloride, alkyldiamino ether glycin acetate, sodium hypochlorite, acetyl hydroperoxide, sodium sesqui-carbonate, silica, povidone-iodine, sodium hydrogen carbonate. In addition, high density carbonate spring may be added (examples of organic components are sulfate, carbonate, or sodium dichloroisocyanurate).

At the bottom center of the generator main body 41 (the liquid storage 42), the nozzle 43 is placed. This nozzle 43 upheaves from the bottom of the liquid storage 42, and is shaped to be almost a circular cone squeezed toward an upper side of the generator main body 41. The nozzle 43 is connected, at its base end, to a gas discharge pipe 45A of the gas supply means connecting portion 45 to enable to discharge gas from its point open 43A.

The liquid suction pipe 44A is formed between the outer circumference of the nozzle 43 and the liquid suction pipe forming member 44 of the almost circular cone being larger by one turn than the nozzle 43. That is, as shown in FIG. 2, by positioning as covering the liquid suction pipe forming member 44 over the nozzle 43, the liquid suction pipe 44A is defined between the outer circumference of the nozzle 43 and the inner circumference of the liquid suction pipe forming member 44. Although having omitted to show, since a minute nail shaped projection is provided at a base end (the lower part of the almost circular cone part) of the liquid suction pipe forming member 44, a space is defined on the bottom between a base of the liquid suction pipe forming member 44 and the bottom of the liquid storage 42, and from this space the liquid stored in the liquid storage 42 is sucked up by the liquid suction pipe 44A. In addition, the front end 44B of the liquid suction pipe forming member 44 opens nearly the point open 43A of the nozzle 43 , and the liquid sucked up by the liquid suction pipe 44A collides with the gas flow discharged from the nozzle 43.

The gas supply means connecting portion 45 is a connecting portion which can attach a gas bomb 31 preferably by one-touch. Although omitting illustration, the gas supply means connecting portion 45 has inside a regulator for enabling to adjust a gas flowing amount supplied from the gas bomb 31. The gas flow from the gas bomb 31 is diverged into two branches (diverging part 45B in FIG. 2) within the gas supply means connecting portion 45, one being supplied to the nozzle 43 while the other being supplied to a later mentioned gas inlet 47. By the way, herein shown is an example where the gas supply means connecting portion 45 is furnished with a dial switch 451 and a residual gage 452 (refer to FIG. 1). By turning the dial switch 451, ON/OFF of the gas supply and a supplying amount control are made available. The residual gage 452 indicates a gas remaining amount of the gas bomb 31.

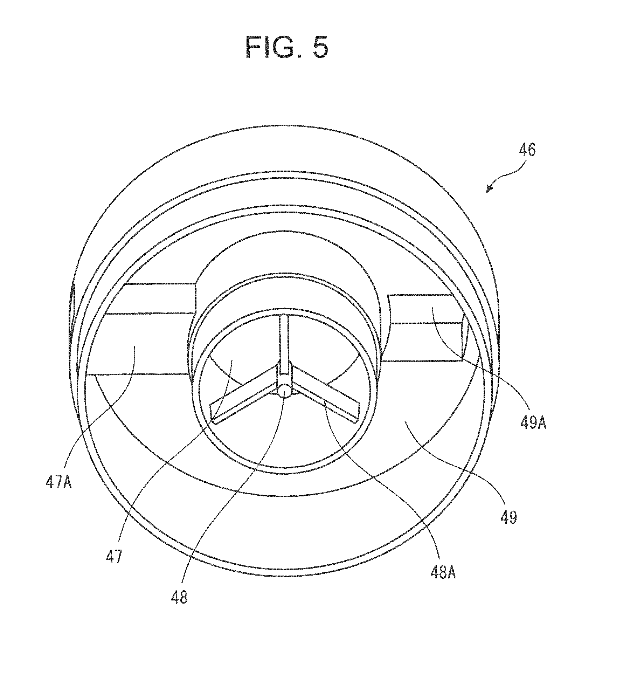

The cover part 46 is a member which is attached on the upper part of the generator main body 41 and introduces the generated gas mist into an inhalation mask 51. Further, if supplying gas into the generator main body 41 separately from the nozzle 43, the member 46 heightens supplying pressure of the gas mist into the halation mask 51. Details of the cover part 46 are shown in FIG. 5. FIG. 5 is the perspective view under the condition when looking the cover part 46 from its bottom side.

The cover part 46 has a gas inlet 47 which introduces gas into the generator main body 41 and makes air current for discharging the gas mist, a baffle (collision member) 48 disposed at a position in opposition to the point open 43A of the nozzle 43, and a gas mist outlet 49 which collects the gas mist and discharges into the halation mask 51.

The gas inlet 47 is a substantially L-shaped pipe hole for guiding gas from the outside of the cover part 46 until the periphery of the nozzle 43 of the generator main body 41, and the gas diverged by the gas supply means connecting portion 45 is supplied from this place into the generator main body 41. The gas inlet 47 is furnished on its upper part with a gas introducing mouth 47A connected via a tube or the like to the gas supply means connecting portion 45. The gas introducing mouth 47A may be furnished with an air hole for taking in outside air, though not illustrating here. It is sufficient to dispose a flow control (for example, valve) 45C for controlling a gas supply amount to the gas inlet 47 as shown in FIG. 2.

The baffle 48 is placed by a baffle supporter 48A in the vicinity of the lower end of the gas inlet 47. By the way, there is shown the example of the baffle 48 being secured to the cover part 46, but it may be disposed toward the side of the generator main body 41.

A gas mist outlet 49 is a donut shaped space formed around the cylindrical gas inlet 47 by the side and the upper part of the inside of the cover part 46. As having mentioned above, preferably, the gas inlet 47 is cylindrically shaped and the gas mist outlet 49 is donut-shape, but not necessarily limited thereto. Thus, by not partitioning but widening the space of the gas mist outlet 49, the mist can be smoothly discharged and prevented from liquefaction. Further, since the structure is simplified thereby, the cover part 46 is heightened in washing efficiency and can be served hygienically.

The gas mist is generated owing to the nozzle 43 and baffle 48, and driven to the gas mist outlet 49 by gas from the gas inlet 47. The gas mist outlet 49 leads the gas mist to an inhalation mask 51. At the upper part of the gas mist outlet 49, there is provided a gas mist outlet 49A connected to the inhalation mask 51.

The inhalation mask 51 is such an inhalation member having a shape covering a user's respiratory organs (herein, the nose and mouth) for the user to easily breath the generated gas mist. The inhalation mask 51 is connected to the gas mist outlet 49 via the connecting portion 52, and the user breathes the gas mist from the inhalation port 53. The inhalation mask 51 is preferably formed with an aperture 54.

In the following, explanation will be made to one example of procedures for carrying out inhalation of the gas mist with the gas mist inhaler 10 of the above mentioned first embodiment.

At first, the sealed generator main body 41 is opened, and the gas supply means connection portion 45 and the cover part 46 are set to accomplish the gas mist generator 40. Subsequently, the gas bomb 31 is set to the gas supply means connection portion 45. When turning ON a dial switch 451 of the gas bomb connecting portion 45, gas starts to go into the nozzle 43 and the gas inlet 47. Incidentally, the dial switch 451 can adjust a gas flowing amount.

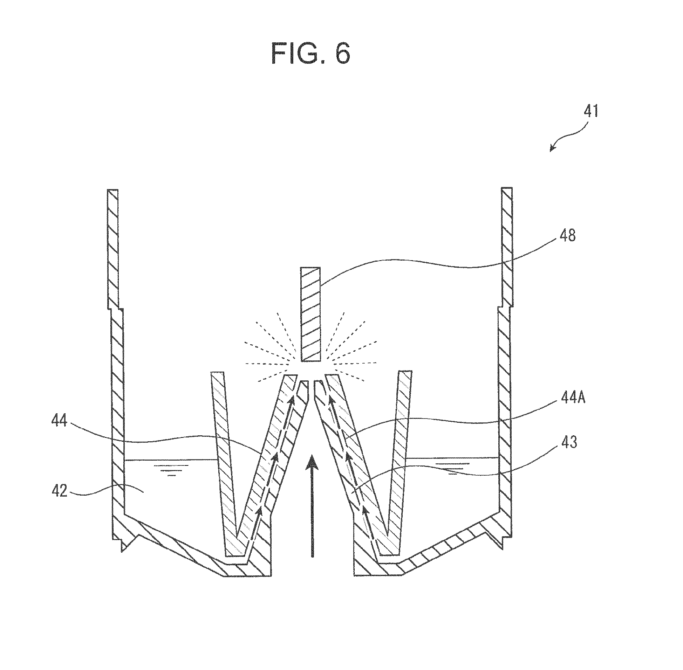

When gas is supplied into the nozzle 43, since the nozzle 43 is reduced in a diameter toward the front end as shown in FIG. 6, the gas increases the flowing speed and is exhausted. The liquid is sucked up within the liquid suction pipe 44A owing to negative pressure caused by air flow at this time, is blown up by gas at the front end portion 44B of the liquid suction pipe 44A, and collides with the baffle 48, so that the mist is generated. Desirably, the diameter of the mist generated by this collision is fine, and concretely, best is not larger than 10 µm. The thus finely pulverized mist can display effects of minus ion.

The gas is further supplied into the generator main body 41 also from the gas inlet 47, and heightens the exhausting pressure of the generated gas mist. The gas mist is exhausted from the gas mist outlet 49 into the inhalation mask 51. The gas mist exhausted in the inhalation mask 51 can be inhaled from an inhalation port 53.

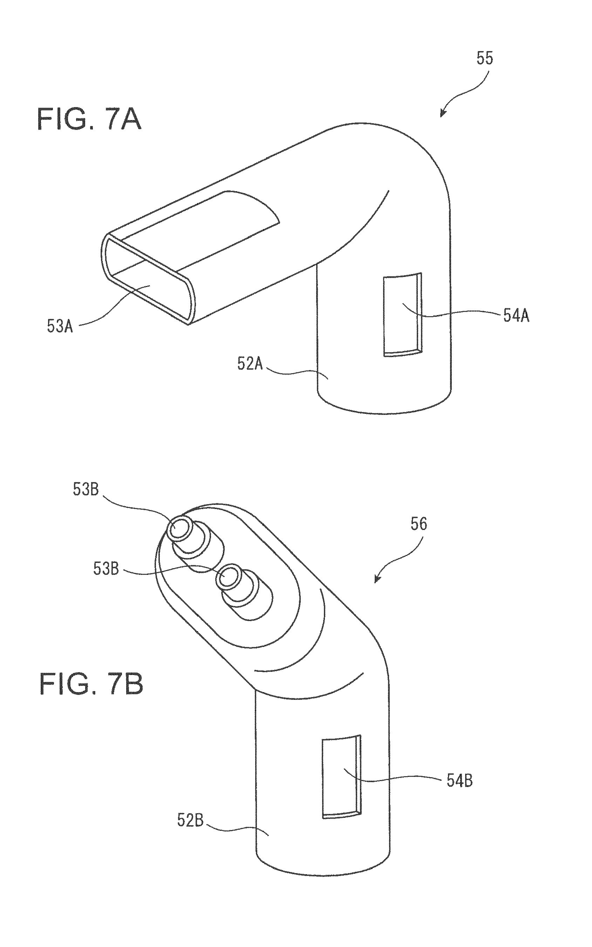

The above reference has showed, as the inhalation member, the example of using the inhalation mask 51 of a type covering the nose and the mouth, and other various types using the inhalation members are available. FIGs. 7A-7B show other examples of the inhalation members.

FIG. 7A is a mouth mask 55 of a mouth piece type used for inhaling from the mouth only. At the connecting portion 52A, this is connected to the gas mist outlet 49, are the user inhales the gas mist into the mouth from the inhalation port 53A. The mouth mask 55 is especially suitable to moderate and cure symptoms of the throat.

FIG. 7B is a nose mask 56 of a nose piece type used for inhaling from the nose only. At the connecting portion 52B, this is connected to the gas mist outlet 49, and the user inhales the gas mist into the nose from the inhalation port 53B. The nose mask 56 is furnished with an open 54B, and is especially suitable to moderate and cure allergic rhinitis of the nose.

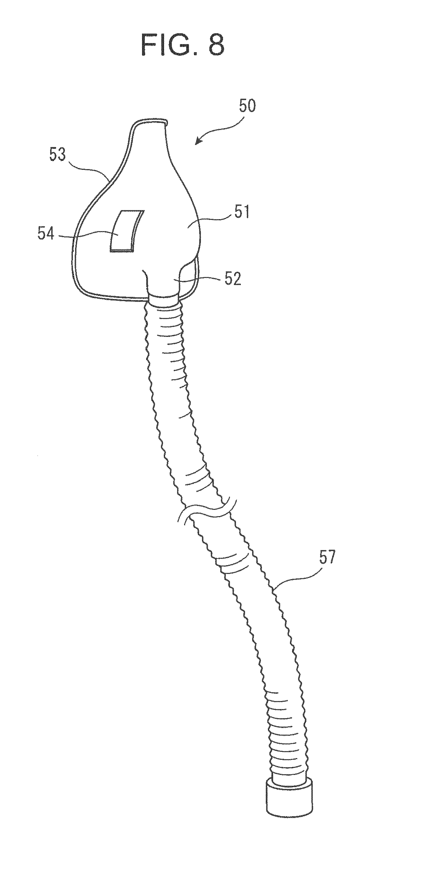

The above reference has shown the example where the gas mist outlet 49 is directly connected to the connecting portion 52 of the inhalation member 50, and as shown in FIG. 8, the gas mist outlet 49 may be also connected to the inhalation mask 51 via the gas mist supply pipe 57. For such a case, the inside of the gas mist supply pipe 57 is furnished with a check valve for preventing back flow of the gas mist, though not illustrated. The gas mist supply pipe 57 may be provided with a filter for removing surplus liquid drops attached to the inside of the pipe, though not illustrated.

Further, if the gas mist supply pipe 57 is composed wholly or partially with a cornice shaped and soft pipe of large diameter as shown in FIG. 8, it is freely bent and contracted so that the user's action is not limited. In addition, even if the gas mist flowing in the gas mist supply pipe 57 becomes liquefied, the cornice can remove the liquid owing to its concave and convex parts.

### [Second Embodiment]

The above first embodiment has shown the example as the gas supply means 30 using the gas bomb 31 of the small sized cartridge system, and the present embodiment will show an example using a gas supply device of a stationary type.

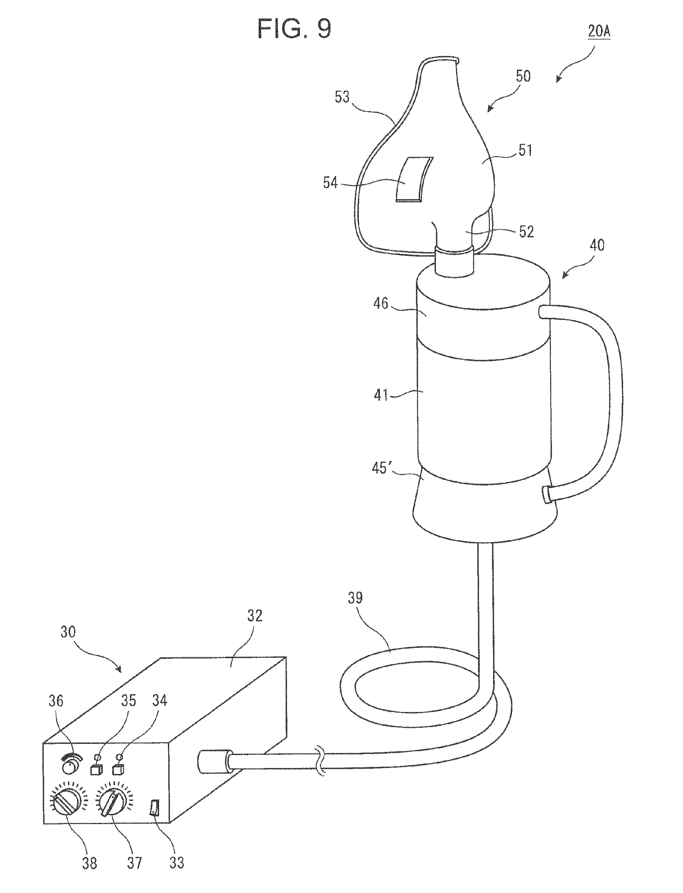

FIG. 9 is the generally schematic view of the gas mist inhaler depending on the second embodiment of this invention. As to the same parts as those of the embodiment shown in FIGs. 1 to 8, the same numerals will be given, and detailed explanation will be omitted.

As shown in FIG. 9, the gas mist inhaler 20A of this embodiment has a gas supply device 32 as the gas supply means 30, the gas mist generator 40 as the gas mist generating means and the inhalation mask 51 as the inhalation member 50.

The gas supply device 32 is the stationarily typed device for supplying gas (oxygen, carbon dioxide or the mixed gas of oxygen and carbon dioxide) into the gas mist generator 40 at predetermined pressure. Its inside is built-in with the gas bomb (not shown), and may be built-in with a compressor, otherwise may be connected to an external gas bomb.

The gas supply device 32 is, for example as shown in FIG. 9, provided with an power supply switch 33, oxygen supply ON/OFF switch 34, carbon supply ON/OFF switch 35, gas mixing ratio setting part 36, OFF timer (gas supply time setting part) 37, and gas supply pressure adjusting part 38. That is, in the present embodiment, the gas supply means connecting portion has neither the dial switch nor the residual gage (the gas supply means connecting portion 45'), but various controls are carries out by the gas supply device 32. The gas supply device 32 and the gas mist generator 40 are connected by the gas supply pipe 39.

The gas mixing ratio setting part 36 is so composed as to regulate arbitrarily the mixing ratio of carbon dioxide and oxygen. It is thereby possible to reply at will to the user's requests, for example, for lightening asthma or recovering fatigue such as setting the oxygen mixing ratio to be much, or for accelerating a blood circulation such as setting the carbon dioxide mixing ratio to be much. Further, the OFF timer 37 is for setting the gas supplying time, and when a set time passes away, the gas supplying is automatically stopped. The gas supply pressure adjusting part 38 can set the gas supplying pressure arbitrarily. Since the gas supplying time or pressure are possible thereby, its using scope can be broadened.

Incidentally, similarly to the first embodiment, herein illustrated is an example where gas supplied from the gas supply device 32 is diverged into two at the gas bomb connecting portion 45, and instead of this example, such a structure is also enough in which gas is directly supplied into the nozzle 43 and the gas inlet 47 from the gas supply device 32. In such a case, the same gas may be supplied in the nozzle 43 and the gas inlet 47, or different gases may be supplied.

### [Third Embodiment]

The above second embodiment has shown the example where one inhalation member 50 is connected with one gas mist generator 40, and the present embodiment will explain a structure of connecting a plurality of inhalation members.

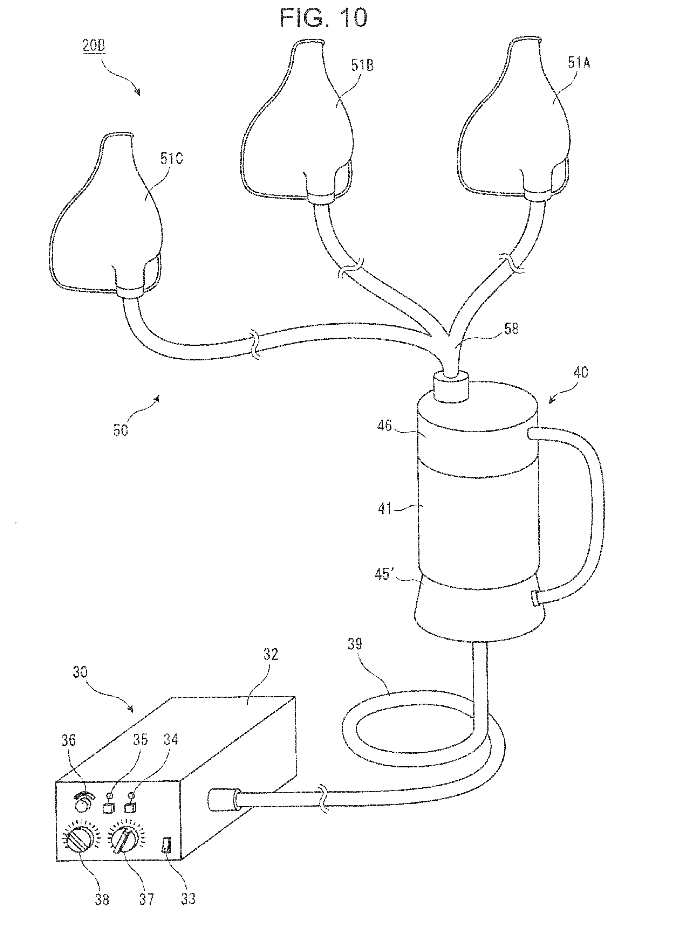

FIG. 10 is the generally schematic view of the gas mist inhaler depending on the third embodiment of this invention. As to the same parts as those of the embodiment shown in FIGs. 1 to 9, the same numerals will be given, and detailed explanation will be omitted.

As shown in FIG. 10, the gas mist inhaler 20B of this embodiment has the gas supply device 32 as the gas supply means 30, the mist generator 40 as the gas mist generating means, and plural inhalation members 50 (herein, as example, plural inhalation masks 51A to 51C).

The present embodiment equips a gas mist supply pipe 58 diverging into a plurality of pipes (herein, three) between the gas mist generator 40 and a plurality (herein, three) of inhalation members 50. It is thereby possible to connect the plurality (herein, three) of inhalation members 51A, 51B, 51C to one gas supply device 32 and the gas mist generator 40. At this time, the gas supply device 32 adjusts the supplying pressure by the gas supply pressure adjusting part 38 such that a gas inhalation can be performed optimally in each of the plural inhalation members 51A, 51B, 51C.

The present embodiment has illustrated the example using the gas mist supply pipes 58 diverging into the plurality of pipes, and the gas mist outlet 49 may provide plural outlets, each of which is connected with the gas mist pipe, or provide diverged plural pipes on the way of the ordinary gas mist pipes for supplying the gas mist into the plural inhalation members.

As having above mentioned, according to the gas mist inhaler of the present invention, in addition to ordinary effects of the inhaler, and owing to the physiological action of the gas mist, not only permeating the liquid medicine into the upper and lower airways of the living organism, but also activating a blood flow around a diseased part, the invention can display effects such as rapidly moderating an inflammation or increasing immunological force.

As having above mentioned, according to the gas mist inhaler of the present invention, the liquid storage being one part of the gas mist generator is made displaceable and replaceable with the other liquid storage, and the liquid storage after having been used is made disposable, and enables reduction in costs together with paying attention to hygiene. Thereby, with the simple structure, by the physiological action of the gas mist, not only permeating the liquid medicine into the upper and lower airways of the living organism, but also activating a blood flow around a diseased part, the invention can display effects such as flourishing the blood flow of the disease, rapidly moderating an inflammation or increasing immunological force.

The above references have explained the embodiments of the invention, but are not limited thereto, and so far as not deviating from the subject matter of the invention, various kinds of embodiments are, of course, available.

### INDUSTRIAL APPLICABILITY

The present invention relates to the gas mist inhaler for carrying out oral inhalation of the gas mist into the living organism, the gas mist having been prepared by pulverizing and dissolving oxygen, carbon dioxide or the mixed gas of oxygen and carbon dioxide, and liquid of medicines with industrial applicability.

### EXPLANATION OF THE REFERENCE NUMERALS AND SIGNS

10: gas mist inhaler
20A: gas mist inhaler
30: gas supply means
31: gas bomb
32: gas supply device
33: power supply switch
34: oxygen supply ON/OFF switch
35: carbon dioxide supply ON/OFF switch
36: gas mixing ratio setting part
37: OFF timer (gas supply time setting part)
38: gas supply pressure adjusting part
39: gas supply pipe
40: gas mist generator
41: generator main body
41A, 41B: plug
41C: film
42: liquid storage
43: nozzle
43A: point open
44: liquid suction pipe forming member
44A: liquid suction pipe
45, 45': gas supply means connection portion
45A: gas discharge pipe
45B: branch part
45C: flow control
451: dial switch
452: residual gage
46: cover part
47: gas inlet
48: baffle (collision member)
48A: baffle supporter
49: gas mist outlet
50: inhalation member
51, 51A, 51B, 51C: inhalation mask
52, 52A, 52B: connecting part
53, 53A, 53B: inhalation port
54, 54A, 54B: open
55: mouth mask
56: nose mask
57: gas mist supply pipe
58: gas mist supply pipe

## Claims

1. A gas mist inhaler, comprising:
a gas supply means of supplying oxygen, carbon dioxide, or a mixed gas (called as "gas" hereafter) of oxygen and carbon dioxide;
a gas mist generating means which has a connecting portion connected to the gas supply means, a liquid storage of storing a liquid, a nozzle supplied with the gas, a liquid suction pipe of sending the liquid to the tip of the nozzle, a collision member of colliding the liquid blown up by a gas stream from the nozzle therewith to convert it into a mist (called as "gas mist" hereafter) by pulverizing and dissolving these liquid and gas, a cylindrical gas inlet supplied with the gas and leading the gas till the upper portion of the nozzle, and a donut-shaped gas mist outlet collecting and discharging the gas mist; and
an inhalation member connected to the gas mist generating member and having an inhalation port for enabling to inhale the gas mist into a living organism,
wherein, with respect to the gas mist generating means, at least the liquid storage is replaced by another liquid storage.

2. The gas mist inhaler as set forth in claim 1, wherein the gas mist generating means has an air hole for taking in outside air.

3. The gas mist inhaler as set forth in claim 1, wherein the inhalation member to have a further opening for taking in outside air.

4. The gas mist inhaler as set forth in any one of claims 1 to 3, wherein the gas supply means is a gas bomb of a cartridge system.

5. The gas mist inhaler as set forth in any one of claims 1 to 4, wherein the gas supply means has any one or plurality of a gas supply time setting portion, a gas supply pressure adjusting portion and a gas mixing ratio setting portion.

6. The gas mist inhaler as set forth in claim 5, wherein the gas mist generating means supplies the gas mist into the plural inhalation members.

7. The gas mist inhaler as set forth in claim 1, wherein the above mentioned liquid is any one or plural combination of water, ionic water, ozone water, physiological salt solution, purified water, or sterilized and purified water.

8. The gas mist inhaler as set forth in claim 7, wherein the above mentioned liquid further contains any one or plural combination of menthol, vitamin E, vitamin C derivative, retinol, anesthetic agent, cyclodextrin, photo catalyst, complex of photo catalyst and apatite, hyaluronic acid, coenzyme Q10, seed oil, propolis, ethanol, chlorhexidine gluconate, amphoteric surface active agent, benzalkonium chloride, alkyl diamino etherglycine acetate, sodium hypochlorite, peracetic acid, sodium sesquicarbonate, silica, povidone-iodine, sodium hydrogen carbonate, carbonate spring agent of high density, anti-allergic agent, anti-inflammatory agent, anti-febrile agent, anti-fungus agent, anti-influenza virus agent, influenza vaccine, steroid agent, anti-cancer agent, or anti-hypertensive agent.

9. The gas mist inhaler as set forth in claim 1, wherein a size of the mist supplied from the gas mist generating means into the inhalation member is not larger than 10 µm.

10. The gas mist inhaler as set forth in claim 1, wherein the gas mist generating means has a gas mist supply pipe for supplying the gas mist into the inhalation member, and
the gas mist supply pipe is furnished with a filter for removing liquid drops attached to the inside of the pipe.

11. The gas mist inhaler as set forth in claim 1, wherein the gas mist generating means has a gas mist supply pipe for supplying the gas mist into the inhalation member, and
the gas mist supply pipe is composed of a cornice shaped pipe over a whole or at one part of the gas mist supply pipe.

12. The gas mist inhaler as set forth in claim 1, wherein the gas mist generating means has a gas mist supply pipe for supplying the gas mist into the inhalation member, and the gas mist supply pipe is provided with a check valve.

13. The gas mist inhaler as set forth in claim 1, wherein the removable part of the gas mist generating means is in advance sterilized.
